# EUROPEAN PATENT SPECIFICATION

(11) **EP 2 749 650 B1**
(45) Date of publication and mention of the grant of the patent: **06.03.2019**
(21) Application number: 12199417.2
(22) Date of filing: 27.12.2012
(51) Int. Cl.: C12P 7/62, C02F 3/02, C02F 3/12

(54) **Method for producing polyhydroxyalkanoates by microorganisms**
Verfahren zur Herstellung von Polyhydroxyalkanoaten durch Mikroorganismen
Procédé de production de polyhydroxyalcanoates par des micro-organismes

(43) Date of publication of application: 02.07.2014
(73) Proprietor: Veolia Water Solutions & Technologies Support, 94417 Saint-Maurice Cedex (FR)
(72) Inventor: Albuquerque, Maria, 78780 MAURECORT (FR); Deleris, Stéphane, 78450 VILLEPREUX (FR); Uribelarrea, Jean-Louis, 31400 TOULOUSE (FR); Paul, Etienne, 31290 MONTCLAR LAURAGAIS (FR); Grouseau, Estelle, 31000 Toulouse (FR)
(74) Representative: Vidon Brevets & Stratégie

(56) References cited:
- US-A1- 2007 119 763
- US-A1- 2009 317 879
- INCE O ET AL: "Effect of nitrogen deficiency during SBR operation on PHA storage and microbial diversity", ENVIRONMENTAL TECHNOLOGY (UNITED KINGDOM) 20120801 TAYLOR AND FRANCIS LTD. GBR, vol. 33, no. 16, 1 August 2012 (2012-08-01), pages 1827-1837, XP008162161, ISSN: 0959-3330
- SERAFIM LUISA S ET AL: "Optimization of polyhydroxybutyrate production by mixed cultures submitted to aerobic dynamic feeding conditions", BIOTECHNOLOGY AND BIOENGINEERING, WILEY & SONS, HOBOKEN, NJ, US, vol. 87, no. 2, 20 July 2004 (2004-07-20), pages 145-160, XP002505409, ISSN: 0006-3592, DOI: 10.1002/BIT.20085 [retrieved on 2004-06-16]
- PUNRATTANASIN W ET AL: "Aerobic production of activated sludge polyhydroxyalkanoates from nutrient deficient wastewaters.", WATER SCIENCE AND TECHNOLOGY : A JOURNAL OF THE INTERNATIONAL ASSOCIATION ON WATER POLLUTION RESEARCH 2006, vol. 54, no. 8, 2006, pages 1-8, XP002697392, ISSN: 0273-1223
- ALBUQUERQUE M G E ET AL: "Mixed culture polyhydroxyalkanoate (PHA) production from volatile fatty acid (VFA)-rich streams: Effect of substrate composition and feeding regime on PHA productivity, composition and properties", JOURNAL OF BIOTECHNOLOGY, ELSEVIER SCIENCE PUBLISHERS, AMSTERDAM, NL, vol. 151, no. 1, 10 January 2011 (2011-01-10), pages 66-76, XP027578401, ISSN: 0168-1656 [retrieved on 2010-10-27]
- H Shimizu ET AL: "Maximum production strategy for biodegradable copolymer P(HB-co-HV) in fed-batch culture of Alcaligenes eutrophus", Biotechnology and bioengineering, 5 March 1999 (1999-03-05), pages 518-525, XP055457236, UNITED STATES Retrieved from the Internet: URL:http://onlinelibrary.wiley.com/doi/10. 1002/(SICI)1097-0290(19990305)62:5%3C518:: AID-BIT3%3E3.0.CO;2-6/epdf

## Description

### 1. Field of the invention

The invention relates to a process for selecting polyhydroxyalkanoate (PHA) producing microorganisms from a natural source comprising a variety of microorganisms. The invention also relates to a process for producing PHAs by such selected microorganisms.

### 2. Prior art

Plastics are manufactured by the polymerisation of organic monomers, most commonly derived from petrochemicals. However, oil is becoming rare, plastic manufacturing contributes to greenhouse gas emissions and climate change and furthermore the significant accumulation of plastics in the environment, which are highly recalcitrant to natural degradation, represents a significant concern. Therefore, alternative processes for the production of bioplastics have been developed. One of these alternative processes relates to the production by microorganisms of biobased polymers, like polyhydroxyalkanoates (PHAs), which can be subsequently used as raw materials for bioplastic production. PHA producing microorganisms include wild type natural PHA producers (such as *Cupriavidus necator*), recombinant organisms such as modified *E. coli,* and even microbial consortiums such as those present in biological wastewater treatment systems.

Under unbalanced growth conditions, bacteria store the excess carbon in the form of intracellular reserves by synthetizing PHAs. The intracellular PHA granules can then subsequently be used as a source of carbon and energy by the cells. Such unbalanced conditions can be created by setting the flux of carbon uptake in excess relative to cell growth rate, resulting of nutritional or electron acceptor limitation or lack of intracellular growth requirements (after exposure to long famine periods).

PHAs are a family of linear polyesters of hydroxyalkanoate monomers that display different and interesting thermal and mechanical properties according to their composition. In particular, PHA composition notably depends on the carbon source used as substrate for PHA accumulation and on the bacterial strain. Among others, they are thermoplastic, ductile, more or less elastic, UV stable and show a low permeation to water and air. Thus, PHAs seem to be a very promising solution for the replacement of conventional polyolefins like polypropylene.

Bioplastics can be defined as biobased, i.e. made from renewable and natural raw material, and/or biodegradable plastics. Some currently available bioplastics are biobased without being biodegradable (eg. starch based polymers with petrochemically derived grafts), whereas there are also synthetic based polymers, which can be modified to meet biodegradability standards. PHAs have the advantage of being both biobased and biodegradable, thus allowing for a closed loop production-use-disposal life cycle.

Moreover, as they are both biobased and biodegradable, PHAs allow a short product life cycle: ideal for single use applications in which the product becomes contaminated (eg. waste collection) or applications in direct contact with natural environments. Their production can be considered sustainable. For all these reasons, PHAs arose a great interest of the academic community and the chemical industry.

Two main strategies have been proposed so far for microbial PHAs production and can be classified as:
- the industrial biotechnology approach using pure culture systems of an efficient PHA producing strain, either wild type or genetically modified, and usually costly single carbon substrates (such as glucose, sucrose or corn steep liquor); and
- the environmental biotechnology approach using mixed culture systems, or microbial consortiums composed of various strains of microorganisms, selected from a natural source to be enriched in PHA-storing organisms. These strains are able to use complex feedstocks (eg. industrial wastes and by-products, particularly in the form of liquid effluents) after a pretreatment stage. Usually, the pretreatment stage is an acidogenic fermentation step in order to enrich the effluents in volatile fatty acids (VFAs), which are adequate precursors for PHA storage) as source of carbon for PHA accumulation.

An example of pure culture system is disclosed in patent application US-5871980-A. This application relates to a method for producing PHA by *Alcaligenes eutrophus* cultivation in fed-batch reactor. The discontinuous fed-batch process usually comprises two consecutive phases:
1) a phase for cell growth maximization (under unlimited growth conditions), in order to increase the cell density and thus maximise productivity,
2) a phase for PHA accumulation under limiting nutrient conditions.

According to this technique, bacteria are cultured under sterile conditions, in order to prevent contamination by other than the PHA-producing strain inoculated and thus maximise PHA yield on substrate, PHA content and also control the PHA quality. Indeed, contamination by non-storing organisms would lead to competition in substrate consumption, dilution of the final PHA per biomass ratio obtained and to heterogeneity in the PHA produced. In this document, the C/P weight ratio ranges from 40-100 during the growth stage and 300 - 600 during the accumulation stage.

Pure culture systems ensure high PHA content in cells and high PHA productivity rate (up to 2,86 g/L/h). However, working in sterile conditions increases dramatically the production costs due to high-energy demands. Furthermore, the carbon feedstocks used also constitute an important cost factor (about 40% of the total PHA production costs) [1]. The average cost of PHA production by these methods is then 4 to 9 times the price of conventional plastics made from petrochemicals. Another disadvantage of this technique is the common use of food crops as carbon feedstock for the PHA accumulation process (eg. corn sugar or rapeseed oil). The use of agricultural based crops dedicated to fuel, chemicals and polymer production can lead to an increasing pressure on natural resources and tougher competition for arable lands. Put together, despite a high technical replacement potential relative to polyolefins [2], PHA produced by pure culture systems are still not cost-competitive against conventional plastics.

To overcome these drawbacks, mixed culture systems, like the feast and famine process, have been proposed. According to a variety of literature sources [3-5], feast and famine systems comprise two steps:
1) a selection step of PHA producing microorganisms from a first inoculum comprising a variety of microorganisms operated under continuous mode, more commonly in a sequencing batch reactor (SBR), and
2) a PHA accumulation step conducted in fed-batch mode using the selected microbial consortium.

The name of the process comes from the selection step, which is achieved by the alternation of feast and famine conditions. Each SBR operation cycle is composed of a feast phase and a famine phase. Each SBR cycle consists in cultivating microorganisms under aerobic conditions in either fermented industrial effluents or medium simulating such effluents. During this phase, called the feast phase, the carbon source (typically volatile fatty acids VFA) is fully available. The feast phase is then followed by a phase during which no external carbon source is available but in which nutrients other than the carbon source are available in non-limiting conditions (famine phase). In other words, the microorganisms use the external carbon source during the feast phase to synthetize PHA and can subsequently use the stored PHA for cell growth and/or maintenance requirements during the famine phase. This selection step aims specifically at selecting PHA storing microorganisms versus non-PHA storing microorganisms. More particularly, the feast and famine conditions imposed result in two competitive advantages for PHA-storing organisms: faster substrate uptake during the feast period due to the possibility to drive the excess carbon uptake toward PHA storage and the possibility to grow on the stored PHA during the famine period. The later advantage relies on excess nutrient conditions in the selection stage, enabling cell growth during famine [6-7].

Within this type of mixed culture systems, there are two types of feast and famine process:
- the conventional type of feast and famine processes wherein the microbial culture is subjected to alternate feast and famine conditions and in which the carbon is limiting relative to the nutrients other than carbon. These systems, though enriched in PHA producing organisms (up to 88% ratio of PHA producers over total bacteria have been referred [6]) are generally relatively diverse in terms of microbial composition, comprising usually both PHA producers and non-producers and also usually comprising more than one PHA producing species [3, 8-9]. Furthermore, some publications [10] report significant variation of the population structure over time of operation: though the PHA storage function is maintained, the relative fraction of the different populations varies.
- feast and famine systems as described in patent application EP 2135954 A1. According to this document, a stronger selection pressure is applied to the biomass during the selection step. In particular, this strong selection stage is achieved by the combination of two added pressures on top of the feast and famine conditions and the excess nutrient to carbon ratio: (1) shortening the feed periods and prolonging the famine phase (obtaining even lower feast to famine ratios than those usually reported for feast and famine systems, ie lower than 0.1) and (2) applying a small number of operation cycles per biomass retention time, which in combination with the long famine phase forces the cell renewal to be fully dependant on PHA synthesis. However, this process still comprises two steps.

In general, feast and famine based processes have considerably lower productivity than pure culture processes due to the need for a two-steps process in which the PHA accumulation step (second step) is inoculated with biomass produced in the first step (the selection step). This means that cell concentration in the accumulation step is limited by cell concentration reached in the selection step. The accumulation step of feast and famine processes is usually operated at much lower cell densities than pure culture processes (up to 6 g/L have been reported for mixed culture processes; in the range of 100 g/L, for pure cultures). The dynamic feeding strategy with alternate feast and famine phases applied to feast and famine selection processes promotes an internal growth limitation (to trigger PHA storage), which results as well in low biomass productivity. Furthermore, the need to maintain a low feast/famine phase length ratio in feast and famine selection processes prevents high loading to be imposed to these systems [9], thereby limiting the resulting biomass concentrations. The low cell densities reported in feast and famine processes ultimately limits the processes global productivities and also results in a higher energy demand for biomass concentration and drying following the accumulation step.

It is also important to consider that in order to maintain a real competitive advantage in feast and famine processes, the PHA stored in cells needs to be effectively used as substrate for biomass synthesis. Therefore, the use of excess nutrients relatively to carbon is required during the selection step. On the contrary, during the accumulation step, nutrient limitation is imposed in order to limit cell growth and maximize the PHA storage response. Thus, the process is necessarily to be run in two subsequent steps.

Moreover, the selection stage requires two reaction phases:
- the feast phase, with substrate uptake and conversion to PHA; and then
- the famine phase, which is usually 3 to 5 times, or even 10 times longer than the feast period. During the famine phase, the PHA is hydrolyzed and used as carbon and energy source for cell growth and/or maintenance.

This means that the process productivity is also constrained by the reaction volume and operating requirements (eg. aeration) required to operate the famine phase.

Another disadvantage, associated with the fact that feast and famine PHA production processes require uncoupling of the microbial selection and PHA production stages, is a lower global yield on substrate. This lower global yield is due to the necessary conversion of a part of the external carbon source to PHA during the feast phase of the selection stage. Therefore, the PHA is converted to biomass during the subsequent famine phase, so that subsequently the produced biomass can be used as a biocatalyst for the conversion of VFA to PHA in the final accumulation stage.

Furthermore, the control of the PHA quality can be made difficult as a large number of various PHA storing strains can be selected and cultivated together, in the same reactor. The degree of diversity of the microbial consortiums selected depends on the efficiency of the selection pressure imposed, which in turn depends on substrate composition as well as on a number of operating parameters used to control feast and famine processes. Relatively diverse populations have been reported for most feast and famine processes, comprising not only the dominant PHA storing population or populations but also a significant non-storing flanking population. These systems have also been demonstrated to sustain a highly evolving population dynamics, which can potentially lead to variability of polymer quality in terms of composition and molecular weight.

The present invention is intended to overcome the disadvantages of the prior art. More specifically, the invention is intended, in at least one embodiment, to provide a more efficient method for selecting a stable PHA producing microbial population on an open culture system.

The invention is also intended to provide, in at least one embodiment, such a method, which is less expensive than the methods of the prior art.

The invention is also intended to provide, in at least one embodiment, a method for producing PHA, which meets, at least in part, the requirements of sustainable development.

### 3. Summary of the invention

These objectives, as well as others that will appear below, are achieved by the invention, which relates to a method for selection and maintenance of a selected microbial consortium for polyhydroxyalkanoates (PHA) production, said microbial consortium being fed with at least one readily biodegradable carbon substrate, comprising a first step of aerobic cultivation of said microbial consortium in a mixed biological reactor according to claim 1.

According to the invention, the specific cell growth rate µ₁ of the microbial consortium is fixed at a target value between 0.01 h-1 and 0.1h-1 in said first step of aerobic cultivation and does not vary more than 50% around the target value, and said first step of aerobic cultivation is performed under nutrient limitation such as the readily biodegradable carbon substrate uptake rate qS₁ is unbalanced with said fixed cell growth rate µ₁. The selection is carried out simultaneously and in the same reactor as said PHA production.

The term of "nutrients" is herein understood to comprise any substance, except the carbon source, that contributes to cell growth.

The term of "catalytic biomass concentration" is defined by the biomass concentration or the suspended solids concentration minus the intracellular PHA content and can be then calculated as follows: catalytic biomass concentration = biomass concentration - intracellular PHA content.

The specific cell growth rate is expressed in "h⁻¹". The readily biodegradable carbon substrate uptake rate qS₁ is the rate at which the biomass consumes the readily biodegradable carbon substrate. Otherwise stated, qS₁ is defined by the substrate quantity consumed by the catalytic biomass per time unit and per biomass concentration mass unit (g of carbon source/ hour/ g of catalytic biomass).

Readily biodegradable carbon substrate can be designated hereafter as "the carbon source", "RBCOD substrate", "the carbon substrate" and "the carbon inflow". The term of RBCOD substrate (Readily Biodegradable Chemical Oxygen Demand") is herein understood to comprise any substrate in water that can be readily and biologically degraded. RBCOD includes a diversity of simple organic carbon molecules such as volatile fatty acids, alcohols and sugars.

The method of the invention allows the selection and the continuous cultivation of a PHA storing microbial consortium in only one step, contrarily to the methods developed in the prior art, thanks to the combination of the nutrient limitation and the tight control of the specific cell growth rate. This method is used to implement a microbial consortium selection process aimed to enrich an open microbial culture in efficient PHA storing organisms. Such selection process will first result in the selection of a simplified and specialized microbial consortium characterized by high PHA storage performance, regardless of the composition of the inoculum used to start up the process. Then, maintaining the same imposed operating conditions will result in a permanent selection and continuous cultivation of the selected PHA-storing population. The selected consortium can subsequently be used as a biocatalyst in a PHA production process.

Applying both growth and nutrient limitations will result in conditions favouring a permanent selection. But it is also compatible in terms of stoichiometric and kinetic performance with a production process as the method of the invention allows operating in a single step culture selection/PHA production process.

Given the strong selective pressure imposed, which ensures a stable microbial consortium under continuous operation, the invention allows operating continuously without the need for sterilization. Therefore, the constraints related to the sterilization are avoided.

Moreover, because the culture selection strategy of the method of the invention is not based on a feast and famine strategy, in which the key element is the observed feast phase/famine phase length ratio, the method of the invention allows operation at higher loading that the mixed culture methods of the prior art. Indeed, as previously mentioned, the mixed culture methods of the prior art have been up to date strongly limited in terms of applied organic loading rate.

Moreover, the method of the invention results in higher productivity that the mixed culture methods of the prior art given that:
(1) the organic loading rate that can be imposed, with direct impact on biomass cell concentration, will not be limited by the need to maintain a low feast phase/famine phase length ratio; and
(2) no reactor volume or aeration requirements to ensure a famine period are necessary. Finally, the high selection efficiency of the method of the invention, resulting in a stable microbial consortium with a narrower microbial structure that usually reported for mixed culture methods of the prior art, results in a more homogeneous PHA product with potential benefits to product quality in subsequent applications.
(3) given the strong selection pressure imposed, the time for culture enrichment is considerably shorter than generally described for feast and famine processes.

The method of the invention provides thus a method for the selection and the maintenance of a stable PHA producing bacterial consortium in one step, therefore sustaining an efficient PHA production process, under non-axenic conditions.

The method for the proposed selection is achieved by imposing a double limitation, on carbon and nutrient, to a continuously operated microbial culture system, which can be inoculated with activated sludge of any origin. This double limitation comprises a cell growth limitation, which can be controlled through the imposed biomass retention time (or cell retention time CRT) and a nutritional limitation. For instance, such a nutritional limitation can be achieved through the control of the ratio of the nutrient uptake to carbon substrate uptake (nutrient/C consumed).

PHA-storing microorganisms subjected to this double limitation will maximise the flow of external carbon substrate directed toward PHA storage. By channelling excess carbon substrate toward PHA storage, PHA-storing organisms will be able to take up the carbon substrate at higher rates than non-storing organisms for which the substrate uptake rate is limited by the imposed specific growth rate and the nutrient limitation. Thus, the high PHA storage response promoted by the imposed double limitation conditions will give PHA-storing organisms a competitive advantage relative to non-storing organisms. Furthermore, since the double limitation conditions imposed favour a high PHA storage response at an imposed specific cell growth rate µ that is consistent with viable cell division, it is possible to continuously subject the open culture consortium to these conditions, thereby resulting in a high and stable selection pressure for efficient PHA storing organisms. Given the high selection pressure imposed, the open microbial consortium will be rapidly enriched in PHA producing organisms, whatever the activated sludge inoculum used to start-up the process.

Furthermore, once the selection is done, i.e when the microbial consortium evolved toward a specific PHA producing culture composed of one or more microbial species, the permanent selection pressure will ensure the stability of the consortium over time. Therefore, the method of the invention allows a stable and efficient PHA production process to be sustained by the selected culture. PHA production can then be achieved directly in the same culture system, which will then be used to sustain a continuous PHA accumulation process with a better PHA yield on carbon substrate and a good productivity.

According to the method of the invention, an aerobic microbial cultivation system inoculated with any diverse bacterial inoculum, under non axenic conditions and fed with a RBCOD substrate influent, can be operated in such a way as to apply a selection pressure aiming at function specialization toward PHA storage. The selection method requires that a double limitation be imposed in said first step of aerobic cultivation, comprising the adequate control of the imposed specific cell growth rate µ1 of microorganisms as well as the adequate control of an imposed nutrient limitation. This double limitation is made to create an unbalance between the RBCOD uptake flux relatively to the nutrient flux required to maintain the said fixed cell growth rate µ1, the first being in excess relative to the later, thus compelling microorganisms to channel external carbon substrate uptake toward PHA storage, thereby conferring a competitive advantage to PHA producing microbial strains.

Preferably, the readily biodegradable carbon substrate is volatile fatty acids (VFA). Volatile fatty acids (VFA) as the preferred carbon source can be obtained through fermentation from wastewater treatment by-products and effluents. They replace the need for high cost substrates. The use of an open culture system and low cost carbon substrates can thus significantly decrease PHA production costs relative to the pure culture methods of the prior art.

In other words, the general principle of the invention relies on the careful choice of cultivation parameters allowing for both PHA producers selection and maximization of PHA production maintaining a viable cell renewal and a cell division rate compatible with the imposed cell retention time (CRT), by creating an unbalance between the carbon substrate uptake flux, preferably a VFA flux, and the flux of nutrient required to maintain the cell growth rate µ1.

By imposing a limitation on both the carbon source and the nutrients, the method of the invention ensures the following mechanisms: (1) the nutrient limitation induces PHA production and storing in microorganisms; (2) the flow of limiting nutrient controls both the growth rate µ1 and the catalytic biomass concentration. Taken together, these factors force the cellular metabolism toward PHA accumulation and carbon depletion, while also generating enough biomass to sustain a continuously growing culture.

Moreover, in carbon limited open culture process, microbial competition is based at least partially on substrate uptake rate. The method of the invention enables the selection of PHA producing microorganisms with high kinetic performance, which are the most efficient for an industrial production process, with high positive impact on process productivity. Furthermore, contrarily to mixed culture systems, wherein a high selection pressure can only be imposed through uncoupling of the culture selection and PHA production stages, the method of the invention displays a high selection efficiency, which can be continuously applied in a single stage process.

This feature differs from the conventional mixed culture system of the prior art in the way that the method of the invention enables the selection of a restricted consortium, composed of only efficient PHA storing strains. Otherwise stated, conventional mixed culture systems of the prior art allow for the selection of a broader variety of PHA storing strains, with a large range of kinetic efficiencies, contained in a consortium of various strains, including non PHA producing strains.

In addition, as the PHA composition depends both on the carbon source supplied to the biomass and on the type of strains, PHA production by a restricted consortium results in a more homogenous PHA composition than obtained with the mixed culture systems of the prior art.

Another parameter of the method of the invention is the fixation of the cell growth rate µ₁, the initial specific growth rate required for the selection or the PHA producing biomass. µ₁ is fixed in order to maintain a residual cell growth and cell division, ensuring the constant renewal of the high kinetic performance PHA storing biomass and their high kinetic performance. It is a fact that the PHA produced quantity increases with the number of PHA storing cells in the reactor (which is in turn dependent on the amount of carbon substrate fed). In a single stage system operated to both ensure a selection pressure and impose operating conditions for an optimized PHA production process, the selection parameters have to be carefully fixed and controlled to allow at the same time (1) a strong selection of the most efficient PHA storing strains, while (2) maintaining a cell proliferation rate resulting in a catalytic biomass concentration consistent with a high process productivity, and (3) at conditions resulting in the optimisation of PHA storage in cells by adjusting the carbon uptake for growth to the total capacity of carbon assimilation.

In other words, carrying out the selection stage simultaneously and in the same reactor as the PHA production stage permits:
- to apply a selection pressure resulting in a restricted consortium of highly efficient PHA storing microorganisms, from any activated sludge inoculum;
- to apply operating conditions maximizing the PHA storage response of the selected microorganisms; and
- the maintain of a residual growth of only the PHAs storing organisms.

In addition, as the method of the invention does not need to be implemented in axenic conditions, PHA is produced at a price, that can challenge other bioplastics currently available (like PLA), and even conventional plastics.

In an advantageous embodiment, the pH value on the aerobic and mixed biological reactor is fixed between 5.5 and 9, and preferably between 6.5 and 8. The pH should be maintained within these values in order to avoid the selection of other microorganisms that do not accumulate PHA.

According to an advantageous solution, the ratio µ1/qS₁ between the specific cell growth rate µ1 and the uptake rate of readily biodegradable carbon substrate qS₁ is comprised in a range between 0.01 and 0.5 g of biomass produced/g of readily biodegradable carbon substrate consumed, preferably between 0.05 and 0.25 g of biomass produced/g of readily biodegradable carbon substrate consumed.

According to an advantageous solution, said first step of aerobic cultivation is carried out in a single stage in a mixed, continuous or semi-continuous biological reactor.

Indeed the method of the invention enables to carry out the selection and the PHA production steps in a single biological reactor, in only one step, contrarily to the process of the prior art.

According to another advantageous solution, said first step of aerobic cultivation comprises further one or more additional stage, each of said additional stages being carried out under more severe nutrient limitation than the previous on, the specific cell growth rate of each stage being lower than the specific fixed cell growth rate of the previous one and each stage being carried out either in a mixed continuous, semi-continuous, batch or fed-batch biological reactor.

In this case, the first stage is the stage that ensures the selection pressure resulting in a stable PHA producing consortium and all subsequent stages are used to maximise PHA content, process productivity or manipulate PHA composition. However, the subsequent stages do not have any impact on the composition of the microbial consortium. It is as well important to note that the aerobic cultivation of these subsequent stages can be carried out either in continuous or batch/fed batch mode. The choice between application of the method of the invention in a single stage process or in a multiple stage process will be based on the target PHA cell content, desired PHA composition, target productivity, and the complexity of the methods used for process control.

Furthermore, the second stage can also be used to control the PHA composition of the produced PHA, since a different impact of nutrient limitation is observed on the HB and HV synthesis rates. Respectively, the use of stricter limitation conditions can be used to control the HV/HB synthesis rate, thus controlling the HV fraction in the P(HB-co-HV) copolymer, with subsequent impact on polymer thermal and mechanical properties which vary with PHA monomeric composition.

In an advantageous solution, the fixation of the cell growth rate, during the first continuous cultivation step, is achieved by the control of the cell retention time (CRT). This feature applies as well when the first aerobic cultivation step is carried out either in a single stage or in two or more stages.

The cell residence time is calculated by the following formula: CRT₁ = 1/µ₁. The CRT can be fixed through the applied dilution rate, which is the ratio of influent flow rate over reactor volume (D₁ = µ₁ = Q/V)), in the case of a continuously operated reactor system like a chemostat. It can be as well fixed through the fixation of the purge rate in the case of systems with uncoupling of the hydraulic retention time and the cell residence time such as biofilm reactors, cell recirculation reactors or sequencing batch reactors. The CRT is defined as the dry mass of solids in the process divided by the dry mass of solids exported from the process per time unit.

Otherwise stated, the aerobic and mixed biological reactor is operated at a fixed cell retention time (eg. for a chemostat, the cell retention time is the reverse of the dilution rate), thus imposing a fixed cell division rate. This promotes microorganisms capable of cell division at nutrient-limiting conditions.

The cell growth rate µ₁ of said microorganism is fixed between 0.01.h⁻¹ and 0.1.h⁻¹, preferably between 0.03.h⁻¹ and 0.08.h⁻¹. Maintaining cell proliferation at a low level, contributes to maximize PHA storage.

The specific cell growth rate µ₁ does not vary more than 50% around said fixed target value and more preferably no more than 20% around said fixed target value. In other words, the specific cell growth rate µ₁ is comprised in a range of [target value -50%; target value +50%] and more preferably, in a range of [target value -20%; target value +20%]. It is important to control tightly the fixed specific cell growth rate in order to maintain a constant selection pressure while enabling the concomitant PHA production and storage by the selected biomass.

In an advantageous solution, growth limitation is achieved by the limitation of at least one nutrient chosen among P, N, O, S, K, Mg, Fe or a combination thereof.

Preferably, the limited nutrient is P and the P/C molar uptake ratio is comprised between 0.0002 and 0.0005 when the specific cell growth rate µ₁ is comprised between 0.05 h⁻¹ and 0.01 h⁻¹.

Preferably, the limited nutrient is P and the P/C molar uptake ratio is comprised between 0.0005 and 0.005 when the specific cell growth rate µ₁ is comprised between 0.1h⁻¹ and 0.05 h⁻¹.

When in conditions of double limitation, this parameter can be controlled by the P/C feed flow molar ratio. P limitation slows down cell growth and triggers PHA storage. The inventors found out that a P/C molar ratio comprised between 0.0002 and 0.005 triggers PHA storage while maintaining a residual growth in the reactor. Moreover, the inventors found out that this particular range of P/C molar ratio gives a PHA storage yield close to the maximum theoretical one. This parameter must be tightly controlled in order to ensure double substrate (carbon and nutrient) limitation conditions. Given the need to simultaneously impose a growth limitation and a nutrient limitation, the nutrient limitation and cell growth limitation will need to be defined in pair, that is, the fixed specific cell growth rate µ₁ and P/C molar ratio will need to be jointly defined. For an imposed specific cell growth rate µ₁ between 0.1 and 0.05 h-1, the P/C molar ratio imposed should be in the range of 0.005 to 0.0005, whereas for a fixed cell growth rate in the range of 0.05 to 0.01, the P/C molar ratio imposed should be in the range of 0.0002 to 0.0005.

It is necessary to rapidly impose the adequate nutrient limiting condition by diluting the nutrient adsorbed to the flocs and the intracellular nutrient concentration.

This is done by initially operating said aerobic cultivation system with an influent carbon inflow but no nutrient inflow. For instance, when the limiting nutrient is phosphorus, about 0.2 g of catalytic biomass must be synthesized per mg of adsorbed P to dilute the phosphorus inflow. In conventional activated sludge (not polyphosphate accumulating sludge), the level of adsorbed P is about 1 mg P/g of volatile suspended solids (VSS). This means that in order to wash out adsorbed P, 0.2 g of new biomass need to be generated per g of biomass inoculated, which means about 1/5 of the cell retention time. On the other hand, to dilute the intracellular phosphorus, P level has to decrease from 2-4% to about 0.5%. The time needed to dilute P levels 4 to 8 times, will be that of 2 to 4 cell doubling times, and will therefore be directly dependent on the imposed cell growth rate. The duration of this period will be dependent on the cell growth rate.

It is crucial to prevent that a too low phosphorus concentration be reached because it can block bacterial growth and make the biological reactor system unstable and at risk of wash out. In one embodiment of the invention, C and P feed flows should be brought separately so that the P feed flow can be initiated as soon as the target intracellular P-limiting concentration is reached. This later point can be determined based on the oxygen uptake rate and pH profile. In an embodiment of the invention, a stepwise decrease of the target P-limiting concentration in the bulk can be imposed. This will help prevent a washout.

In a preferred embodiment, said growth limitation is carried out by the separate addition of the readily biodegradable carbon substrate and the depleted nutrient. More preferably, the composition of the readily biodegradable carbon substrate inflow being uncertain, like with waste-based feedstock, said readily biodegradable carbon substrate inflow can be pre-treated in order to deplete the selected nutrient from it.

In an advantageous embodiment, readily biodegradable carbon substrate effluent can be concentrated previous to its injection in the biological reactor in order to increase the carbon substrate concentration. It allows higher resulting catalytic biomass concentration, thus contributing to improve process productivity.

Preferably, the carbon substrate effluent comprises at least 30% of VFA and more preferably, at least 50% of VFA. More specifically, the total COD of the carbon substrate-rich effluent comprises at least 50% of RBCOD substrate and at least 30% of VFA, preferably at least 50% of RBCOD substrate and at least 50% of VFA. "COD" means "Chemical Oxygen Demand".

VFA can be of various origins, preferably from waste or by-product-based feedstock like industrial effluents, wastewaters, organic fraction of municipal solid waste, leachates from markets and collective restoration facilities, etc. Some of these feedstocks are naturally rich in VFA. However, more commonly these types of feedstocks are characterised by a variety of carbon substrates present. The use of VFA precursors for PHA production from complex waste-based feedstocks usually comprises a stage of biological, physical-chemical conversion of the organic matter present in these streams into VFA. In a variant of the invention, the present invention may comprise a preliminary stage for the conversion of the organic fraction of the waste-based feedstock into VFA. Said preliminary stage can be chosen among acidogenic fermentation under anaerobic conditions and physic-chemical processes such as advanced oxidation process, thermal processes.

In an advantageous embodiment, the method of the invention further comprises a step of continuous monitoring of the PHA content and the PHA composition in said microbial consortium. The continuous monitoring of PHA content can be carried out directly or indirectly by any method know in the art, for instance on-line monitoring through FITR (Fourier Transformed InfraRed) spectroscopy, thermogravimetric analysis (TGA) of dried samples or the monitoring of pH variation.

Since the PHA content attained is a direct result of the selection pressure imposed, monitoring the PHA content can serve as a direct measure of the selection efficiency. A PHA content decrease is a direct indication of a loss of selection pressure due to a change in influent C to selected nutrient ratio, and can thus be quickly detected and resolved. It is indeed an aspect of the invention to provide a tight regulation of the production process in order to constantly maintain high and stable selection efficiency.

In an advantageous embodiment, the method of the invention further comprises a step of monitoring the residual readily biodegradable carbon substrate concentration in the biological reactor. This feature is particularly important when RBCOD substrate is mainly composed of VFA. Indeed, the monitoring of residual VFA concentration helps preventing inhibition by VFA substrates, which in some cases occurs even for very low residual substrate concentrations. This is notably the case for propionic acid, which has a very low inhibition constant.

A substrate (electron donor) accumulation indicates that limitation is too strong, and that the microbial culture can no longer sustain the excess carbon uptake flux. VFA accumulation will lead to cell growth inhibition and decrease of the PHA storage response. Fast detection will allow immediate correction through a control loop, thus maintaining constant selection efficiency. A suitable technique for VFA concentration monitoring comprises pH measurement, Near Infrared Spectroscopy (NIR) analysis, monitoring of the respiration coefficient or oxygen uptake rate.

In a preferred embodiment, the method of the invention comprises further a control loop triggered by a parameter chosen among the readily biodegradable carbon substrate accumulation in said continuous biological reactor, the decrease of PHA cell content, the decrease of PHA cell content to a predetermined value or the combination thereof. In particular, the predetermined value can be the target or desired PHA content to reach. It can also be a minimum PHA content value, which can be chosen according to the desired efficiency of the process. More specifically, VFA accumulation in the reactor signifies that limitation is too strong: the unbalance between uptake and growth being too strong, cells can no longer uptake the inflow of carbon substrate and therefore the unconsumed VFA accumulates in the reactor. On the other hand, if the imposed limitation is not strong enough, PHA storage will not be triggered as effectively, thus a PHA content decrease without VFA accumulation indicates that limitation is insufficient.

One can modify the process parameters (cell dilution rate, cell residence time, stopping feeding of either the nutrient or carbon source effluent) to restart PHA production and storage in cells, prevent inhibition or too strong limitation. This feature participates to the fine regulation of the method of the invention.

For all these reasons, the invention provides a PHA production process that is sustainable, more efficient, easier to implement and less expensive than the methods of the prior art. Put differently, the method of the invention combines the advantages of pure culture systems (high performance: productivity, yield) and mixed culture systems (non-axenic conditions, low cost substrates), while limiting and even avoiding their disadvantages. Therefore, the PHA produced by the method of the invention can challenge other bioplastics and even, oil-based plastics.

The method of the invention can be implemented in a water treatment plant comprising means for injecting a P-source effluent and a readily biodegradable carbon substrate-rich effluent in at least a first mixed biological reactor, and a liquid-solid separator.

The selection and production stage are performed in at least a first mixed continuous or semi-continuous biological reactor. The liquid-solid separator helps to regulate the cell retention time within said continuous and mixed biological reactor in order to uncouple the cell residence time (CRT) from the hydraulic residence time (HRT). Therefore, the cell growth rate can be fixed independently from the loading of VFA and the hydraulic retention time or liquid dilution rate.

The liquid-solid separator can be either downstream or integrated with the continuous and mixed biological reactor. The PHA producing microorganisms pass in the liquid-solid separator by overflow.

The cell residence time is calculated by the following formula: CRT= 1/µ wherein µ is the cell specific growth rate in the reactor. Therefore, the liquid-solid separator helps to the regulation and the bearing of the cell specific growth rate µ.

The water treatment plant further comprises at least one or more additional mixed biological reactor(s), arranged in chain, said one or more additional mixed biological reactor being operated either in continuous, semi-continuous, batch or fed-batch mode, wherein the nutrient limitation in each additional biological reactor is stronger than in the previous one.

Actually, imposing suddenly a too severe limitation can jeopardy cell division and metabolic capacity. Therefore, it can be interesting to increase gradually the nutrient limitation degree in order to maximize PHA storage thereby attaining a higher PHA content in subsequent stages than in the first stage, thus maintaining the first stage as means of maintaining a permanent selection pressure, cell renewal capacity and maximising productivity (higher kinetics obtained at lower PHA content) and using the subsequent stage to maximise PHA content and/or manipulate PHA composition.

It can also help increase system robustness by not operating the selection system too close to strong limitation conditions, which may lead to culture washout. In later stage, this risk is no longer present since these stages are not meant to ensure cell renewal, only maximise PHA cell content in the previously selected consortium.

Additionally, the stepwise increase in degree of imposed limitation can also serve to impose in a second stage a degree of limitation that is not compatible with cell renewal. This subsequent stage can be conducted either in a continuous bioreactor sequentially disposed relative to the first stage or in batch or fed-batch mode. Furthermore, it has been shown that the degree of limitation can be used to manipulate the polymer composition since, given the presence of a VFA precursor to HV synthesis (like propionate and valerate), severe P limitation and even complete depletion leads to higher HV synthesis rate over HB, (thus increasing the HV content of a copolymer). Therefore, in such a configuration, the first stage can be used to maintain high process productivity, in terms of biomass and PHA, while the subsequent stage can be used to maximise PHA content and target specific PHA compositions.

In an alternative variant each additional biological reactor is located between the first continuous and mixed biological reactor and the liquid solid separator, or located downstream the liquid-solid separator.

According to this alternative, each additional biological reactor is connected to a liquid-solid separator. Each liquid-solid separator can be either located downstream or be integrated with each additional biological reactor. A suitable liquid-solid separator can be chosen in the group comprising a sieve, a membrane filter, a settler or a centrifuge.

Preferably, the first continuous and mixed biological reactor and each additional biological reactor is a biological reactor with a cell recirculation loop chosen in the group comprising a chemostat, a chemostat associated to a separator, a membrane biological reactor (MBR), a fixed bed biofilm reactor or a granular reactor. A cell recirculation loop enables the tight regulation of the CRT and then the cell growth rate µ in each biological reactor. More preferably, the first continuous and mixed biological reactor is a chemostat or a chemostat associated to a separator and comprises a cell recirculation loop.

The first continuous and mixed biological reactor and/or each additional biological reactor may comprise means for monitoring the PHA content in microorganisms and/or the residual readily biodegradable carbon substrate concentration. In another variant only the first continuous and mixed biological reactor comprises means for monitoring the PHA content in microorganisms and/or the VFA concentration. The PHA content and/or the VFA concentration are indicators of the cell physiological condition and the performance of the process. At a given specific cell growth rate, a high VFA concentration in the biological reactor indicates that limitation is too severe. A decrease in PHA content, relative to the previous value, without any observed VFA accumulation is an indicator that nutrient limitation should be increased. VFA concentration and PHA content can be monitored by any methods known in the art like pH monitoring, NIR, FTIR and TGA.

The water treatment plant may comprise further means for injecting the readily biodegradable carbon substrate-rich effluent and means for injecting the P-source effluent as separate streams. The plant permits then a better regulation of the carbon source and phosphorus availabilities.

### 4. List of figures

Figure 1 is a diagram of a treatment plant for industrial effluents comprising a one-stage PHA production unit according to the invention.
Figure 2 is a graph representing the results or PHA production related to P limitation.
Figure 3 A-C presents pictures representing the evolution of the microbial consortium in a one stage PHA production unit, from the 7^{th} day of cultivation to day 72.
Figure 3D presents graphs representing the evolution of the microbial consortium in a one-stage PHA production unit.
Figure 4 is a diagram representing the results of PHA production by the method of the invention in a one-stage production unit.
Figure 5 is a schematic representation of a wastewater treatment plant configuration comprising a two-stage PHA production unit.

### 5. General principles

The general principle of the invention relies on the careful choice of cultivation parameters allowing for both PHA producers selection and maximization of PHA production maintaining a viable cell renewal and a cell division rate compatible with the imposed cell retention time (CRT), by creating an unbalance between the carbon substrate uptake flux, preferably the VFA flux, and the flux of nutrients required to maintain the cell growth rate µ₁. The nutrient limitation creates a cellular stress, triggering PHA intracellular accumulation in microorganisms. Whereas the maintained residual cell division rate ensures biomass production.

Otherwise stated, the principle of the invention relies on the application of a permanent selective pressure to an aerobic culture system in order to select only the most efficient PHA storing microorganisms. Contrarily to the methods of the prior art wherein the selection and the production steps are carried out consecutively in different reactors, the selection and production steps are here implemented simultaneously, permanently and in the same biological reactor than the PHA production stage. More specifically, the selection pressure is such that the metabolism is directed to PHA storage while maintaining a residual cell growth and division. The method of the invention maximizes the PHA storing in cells by imposing a residual constant active cell renewal.

These objectives are achieved by the careful choice of parameters, which are:
- the application of a fixed cell specific growth rate µ₁,
- the cultivation in an aerobic and continuous or semi-continuous biological reactor,
- the nutrient limitation and
- the control of all these parameters in order to create an unbalance between the carbon source uptake by the PHA storing cells and their proliferation rate.

An other important parameter is the strict observation of a selection protocol during reactor start-up comprising a non-negligible inoculum concentration, the observation of a nutrient depletion period (in which only the RBCOD substrate is added), the choice of a nutrient limitation trajectory (eg. a step wise decrease of the imposed cell growth rate in parallel with a step wise decrease of P/C feed molar ratio), the selection of the fixed cell residence time and a nutrient/C ratio for subsequent phase of continuous operation.

The invention and the various advantages of it will be more easily understood after reading the following examples given with reference to following figures that diagrammatically show water treatment plants which can be used for the implementation of the method of the invention.

### Example 1 - One stage PHA production system to valorise industrial effluents by the method of the invention.

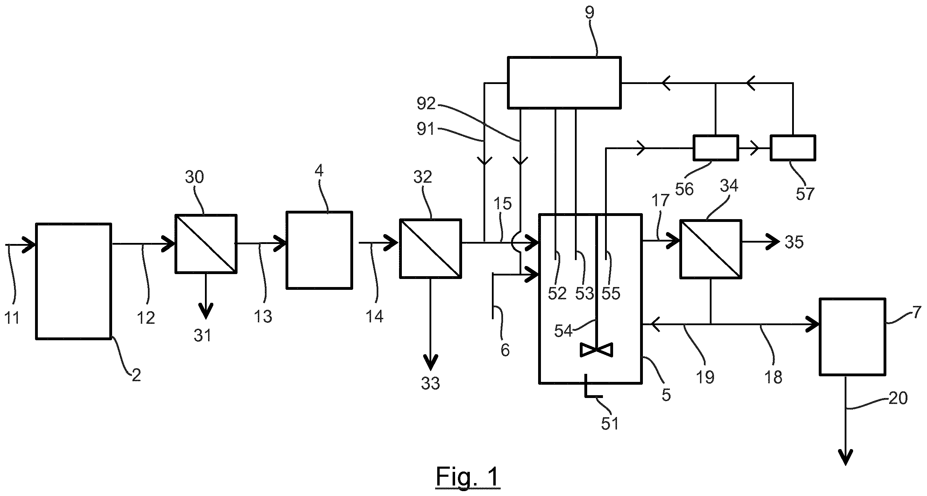

With reference to the figure 1, a water treatment plant for the implementation of the method of the invention is disclosed. Figure 1 shows a diagram or an outline of how the method of the invention works and is not limitative of how the apparatus must be spatially organized.

An industrial effluent of any type 11 enters in a VFA production unit 2. The VFA production unit is a tank wherein VFA are produced by any method known by any person skilled in the art like thermal hydrolysis, oxidation or acidogenic fermentation. The effluent 12 is therefore enriched in VFA. This VFA-rich effluent 12 enters in a liquid-solid separator 30 wherein suspended solids are discarded from the VFA-rich effluent thus producing a clarified VFA rich effluent 13 and a suspended solid stream 31. Liquid-solid separation can be performed by any conventional method: settling, membrane filtration, flocculation and granular or biofilm reactor.

The clarified VFA-rich effluent is submitted to a phosphorus precipitation step in a phosphorus precipitation unit 4. P precipitation can be implemented by any method known of the person of the art like chemical precipitation or ion exchange processes for liquid effluents with little suspended matter. This step allows producing a VFA-rich effluent depleted in phosphorus P. It is then easier to control the composition of the feeding effluents and the double limitation imposed to the microorganisms. A second liquid-solid separation step is carried out in a liquid-solid separator 32, like a settler or a centrifuge, in order to separate the coagulants phosphorus adsorbed on the flocs rejected in a stream 33 from a stream 15, rich in VFA and poor in P.

Both the stream 15 and a P source 6 are added separately in the PHA production unit. The separate addition of the carbon source and the nutrient source makes easier the control of the feeding of the cells and therefore, the control of the imposed double limitation. It is indeed important for the implementation of the method of the invention that the feeding parameters are tightly set and controlled. Preferably, the P-source inflow comes from a natural source, and more preferably from waste-based P recovery process like struvite. The P/C molar ratio of the reactor 1 is fixed and maintained between 0.005 - 0.005.

The PHA production unit is a mixed continuous or semi-continuous biological reactor 5. This reactor is fully aerated by an oxygenation mean 51, like an air blower, and is provided with a mechanical agitator 54, like a propeller. The reactor contains an activated sludge as an inoculum. The cell growth rate µ1 is fixed at 0.05 division/hour.

The continuous and biological reactor 5 comprises as well a temperature sensor 52 and a pH sensor 53. The temperature and the pH sensors (52, 53) are all electronically connected to a control unit 9. A system of gas measurement can also be provided on the outlet 17 of the biological reactor 5 in order to control the cell respiratory coefficient.

The biological reactor 5 includes further a pipeline 17 connecting together the biological reactor 5 and a liquid-solid separator 34. The liquid-solid separator 34 comprises an outlet 35 for removing the exhaust liquid effluent. The main role of the liquid-solid separator 35 is to recycle PHA storing cells toward the biological reactor 5 and control the cell residence time CRT1. As the control CRT1 is inversely proportional to the fixed cell growth rate µ1, the control of said fixed cell growth rate can be achieved by purging the reactor 5 when necessary.

The liquid-solid separation from the settler 34 generates as well a concentrated PHA-rich biomass stream 18, which is directed to a Downstream Processing Unit DSP unit 7, where further concentration (eg. by centrifugation) and drying of the biomass recovered will be carried out. The DSP unit produces a PHA-rich dried biomass 20 from which PHA is recovered by any conventional method like solvent extraction.

It is noteworthy that a part 19 of the concentrated PHA-rich biomass can be redirected to the mixed biological reactor 5 to be enriched in PHA. This cell redirection loop confers more robustness to the PHA production system, rendering it less dependant to the variations of the substrate or biomass composition.

The biological reactor 5 also comprises a purge outlet 55 incorporated in a pipeline. The purge of the biological reactor 1 is controlled to fix the cell specific growth rate by maintaining the cell residence time CRT1 at a fixed value. The cells from the purge are harvested to recover PHA by any conventional method. However, periodic samples are collected from the purge. Biomass is dried in a drier 56 and FTIR or TGA at-line analyses 57 are performed to assess PHA content. The results of the analysis are sent to the control unit 9.

When the biological reactor 5 is operating, pH and temperature are monitored online. The values of these parameters are transmitted to the control unit 9. PHA content is periodically assessed by FITR analysis, twice a day for instance. The results of the FITR analysis can then be entered into the control unit 9, which regulates P and VFA inflows (92 and 91 respectively. Decision rules for the fine regulation of the process are of the following:
1. if estimated PHA content is lower than a desired and minimal PHA content, and simultaneously no VFA accumulation is registered, meaning that a VFA inhibition is not observed, the P/C molar ratio has to be decreased: the VFA-rich inflow has to be increased or the P-source inflow has to be decreased;
2. if estimated PHA content is higher than the target value by a significant degree, this means that the system is at risk of becoming too limited. Therefore, the limitation degree can be reduced by increasing P-source inflow or decreasing the VFA-rich inflow;
3. if pH decreases, there is acid VFA accumulation which means that P/C limitation has to be decreased: P-rich inflow has to be increased or VFA rich inflow has to be decreased. The later option is the best since there was substrate accumulation in the reactor it is better to reduce the incoming flow until the accumulated VFA is reconsumed.
4. if PHA content and the pH value are at least equal to the settled pH and PHA content values, applied conditions are maintained.

### Example 2 - One stage PHA production system to valorise industrial effluents by the method of the invention.

Two open culture systems have been seeded by two different activated sludges coming from a wastewater treatment plant. These open culture systems are similar to the one described in example 1 except the fact that no VFA unit and no P-precipitation unit are provided. Both cultures have been operated under similar conditions. A 5 L bioreactor was operated as a chemostat in order to sustain a cultivation process to carry out the method of the invention.

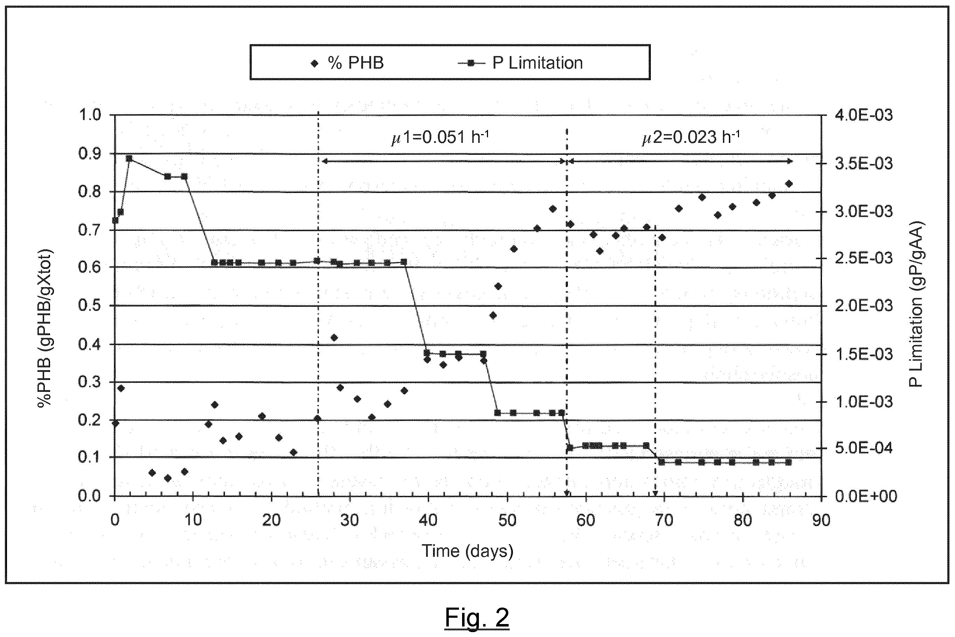

The VFA-rich effluent consists of an acetic acid solution and the P-source stream of a phosphate solution. The cell content of polyhdroxybutyrate PHB, a type of PHA, produced is calculated by the ratio of the weight of PHB stored in cells to the weight of the total biomass (Xtot) in the biological reactor. The P/C limitation is calculated by the ratio of phosphorus consumed to the weight of acetic acid (AA) consumed in the biological reactor. This parameter reflects the unbalance between the AA uptake and the growth rate, limited by a limited P uptake. The results are shown in figure 2.

During the first 72h, a washing of adsorbed and intracellular P was forced on the microbial culture by not feeding any P-source and only the AA feed stream. Then, during the 26 first days, each culture was cultivated in non-growth limiting conditions (imposed growth rate of 0.1 h⁻¹). This preliminary step allows the proliferation of the bacterial population before starting the culture selection process. The cells were submitted to a first degree of P-limitation (decrease from approximately 3.5 x 10⁻³ to approximately 2.5 x 10⁻³ g P/g AA consumed) after 12 days of cultivation in order to slow down their proliferation.

From day 26 to day 58, each inoculum was submitted to a selection protocol intended to demonstrate the selection efficiency of the double limitation method. During this 32 day period, the degree of P limitation was stepwise increased through the decrease of the P/C ratio of the feed (decrease of the P/C uptake ratio from to 2.5 x 10⁻³ to 1.5 x 10⁻³ and then to 1 x 10⁻³ g P/g AA consumed) to enhance the selection pressure on microorganisms while the fixed cell growth rate µ1 was kept at 0,051 division/h⁻¹. We observe that the PHB cell content rapidly increased concomitantly with the degree of P/C limitation, reaching circa 75% of the biomass total weight in 32 days (Figure 2).

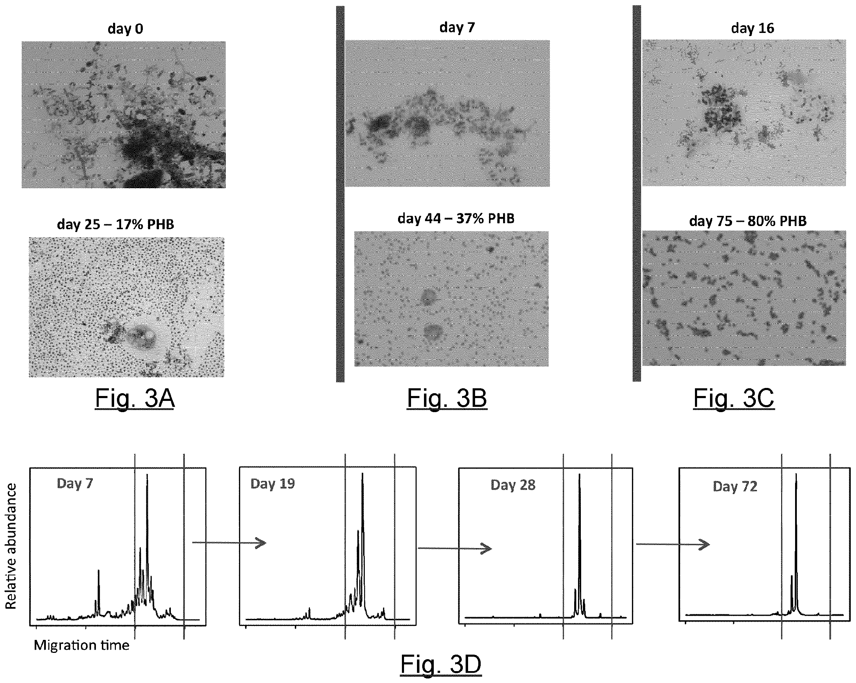

During this period, a simplification of the microbial consortium could be observed through microscopic observations and was confirmed through molecular methods (Figures 3 A-D). More specifically, samples of the bacterial population were taken from the mixed continuous biological reactor at different times of cultivation (day 7 to day 75). These samples were submitted to SCCP analysis, a molecular fingerprint method allowing assessing the number of species present. As shown in figure 3D, the SSCP profiles evolved toward a monoculture. Additionally, the culture was also plated in Petri dishes. Figures 3A-C show the evolution of the bacterial population, which confirm the evolution of a heterogeneous microbial population toward a restricted microbial consortium.

These results were confirmed by the SSCP analysis to be the same found in the bioreactor (Figure 3D). According to these results, the high selection pressure applied to the system resulted in the microbial culture evolving from a diverse consortium to a monoculture (around 99% of the cells belonging to the same bacterial strain). Therefore, the conditions imposed during this period (period I) could be used to sustain a selection method able to simplify an activated sludge diverse inoculum into a monoculture with high function specialization (PHA storage).

Moreover, the PHA content analysis of the bacterial samples reveals that the PHA content in cells is gradually increasing, from 17% of PHB at day 7 to 80% at day 75.

Results obtained allow inferring on the application of the selection method of the invention for either a single stage selection/production process or for a selection process, for which PHA production can be carried out in a subsequent stage (operated either in continuous or fed-batch mode). When the bioreactor was operated at a less stringent P limitation (eg. at 1.5 x 10⁻³ g P/g AA, from days 39 to 48), a PHA cell content around 35% was obtained, along with a simplified culture. The consortium thus selected can then be used to inoculate a subsequent PHA content maximisation stage, said subsequent stage being carried out under a more stringent P limitation). On the other hand, when the bioreactor was operated at a more stringent P limitation (at 1 x 10⁻³ g P/g AA, from days 51 to 58), a PHA content between 65 and 75% was observed. These conditions can thus be used to operate a single stage process for simultaneous culture selection and PHA production.

From day 58 to day 88, cells were submitted to even more stringent double limitation conditions. The specific cell growth rate µ₂ was decreased to 0,023 division/h⁻¹ and the P/C uptake ratio was also decreased in two steps to as low as 0.5 x 10⁻³ and then 0.3 x 10⁻³ g P/g AA consumed. Again the %PHB increased with cell growth and P-limitation, reaching over 80% of PHB of the biomass total weight. The PHA content is fully maximised under these conditions, however, the PHA storage rate is also significantly lower than that observed at the previous growth rate (0.05 h⁻¹), thereby hindering production productivity. Therefore, these set of more stringent growth-limiting and nutrient-limiting conditions could be applied not to a single stage PHA production process, but to the second stage of a double stage process, for instance after applying the less stringent conditions observed during day 26-day 58 for the first stage.

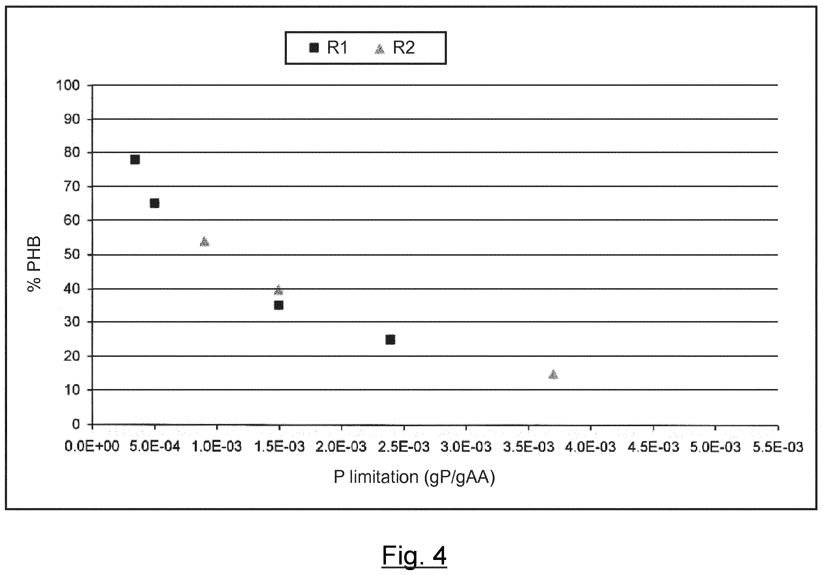

According to these results, the stronger the P limitation is, the more the selected microorganisms produce and store PHA. This observation has been reproduced with the two different inoculums R1 and R2 (Figure 4). Moreover, results obtained show that a stable efficiency is maintained over a 20 day period even under very stringent double limitation conditions, with cell renewal being maintained in the bioreactor as well as a PHA cell content over 75%, showing that once the consortium is selected, a permanent selection pressure is maintaining ensuring the stability of the consortium. As the system evolves into a monoculture or a restricted consortium, the quality of produced PHA is more homogenous and better controlled. Moreover, as only efficient PHA storing microorganisms are selected, the overall efficiency of the process is improved.

At the end of the process, cells in the reactor were recovered and harvested to extract and purify PHB by solvent extraction.

### Example 3 - Two - stage water treatment plant for the implementation of the method of the invention.

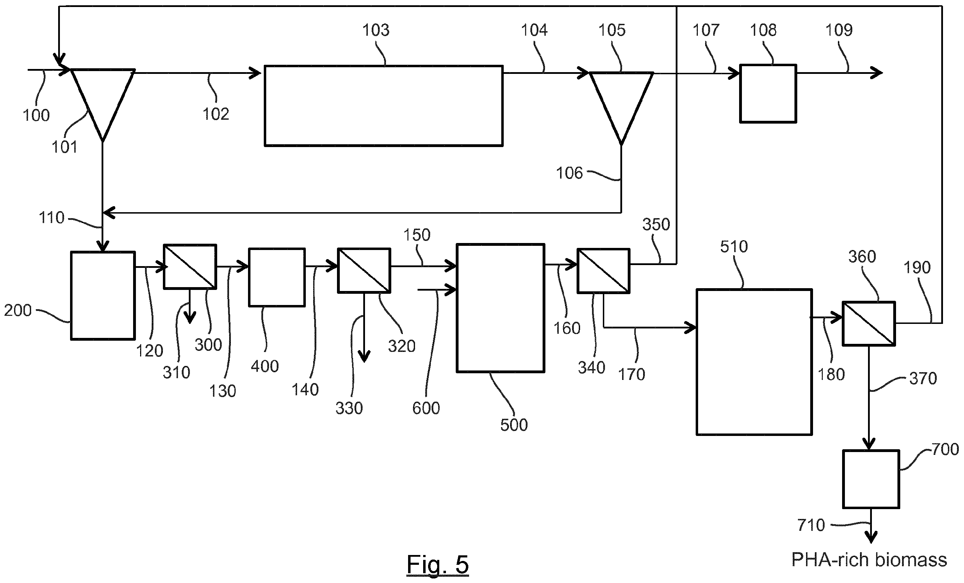

A two-stage PHA production unit integrated in a wastewater treatment plant is outlined at figure 5. It is important to note that a wastewater treatment plant can also integrate a one-stage PHA production unit. Figure 5 shows a diagram or an outline of how the method of the invention works and is not limitative of how the apparatus must be spatially organized.

Wastewater 100 enters in a settler 101 wherein primary sludge 110 and water to be treated are separated. The water to be treated 102 is first subjected to a conventional and biological treatment step 103, by activated sludge for instance. The biologically treated water 104 enters in a second settler 105, wherein the treated water 107 is separated from the secondary sludge 106. The treated water 107 can be then optionally submitted to a tertiary disinfection treatment 108 in order to produce a disinfected treated water 109.

Primary and secondary sludge (110, 106) are recovered and used for acidogenic fermentation for VFA production in a vessel 200 under anaerobic conditions. The VFA flow 120 enters in a liquid-solid separator 300 to separate a clear VFA flow 130 from the biomass and the suspended solids contained in the sludges (106, 110) used for VFA production. Settled biomass and suspended solids are discarded 310. This step of liquid-solid separation prevents the following steps then from a contamination by this biomass. The clarified VFA-rich stream 130 is directed to a P depletion unit 400, for e.g. by a chemical precipitation with MgO₂ or MgCl₂. The VFA rich stream 140 is then submitted to a liquid-solid separation step in a settler 320 in order to separate a clear VFA-rich stream 150, wherein phosphorus has been completely removed, from a stream 330 containing the depleted phosphorus. Optionally, this stream 330 can be used to produce a P-rich source.

The VFA-rich stream depleted from phosphorus 150 is directed to a first continuous and mixed biological reactor 500, which is identical to the biological reactor 5 described in example 1. A pipeline 600 provides the P-rich source. Monitoring and regulation are performed the same way as previously described though at different target operating conditions, since the degree of limitation imposed to the second stage is different than that imposed to the first stage. The regulation is not represented here for clarity reasons. The PHA-rich biomass stream 160 is submitted to a liquid-solid separation step in a settler 340. The liquid phase 350 made of liquid is recycled toward the wastewater treatment line. The PHA-rich biomass stream 170 enters in a second biological reactor 510 to be submitted to a second selection-PHA production step according to the method of the invention.

The second biological reactor 510 is of the same type as the first biological reactor 500 but has a similar or larger volume in order to display a lower dilution rate D₂ from the dilution rate D₁ of the first biological reactor 500 and therefore a lower cell specific growth rate µ₂ from the cell growth rate µ1 of the first biological reactor 500. Preferably, µ₂ is lower than µ₁.

The C/P molar ratio is fixed between 200-2000 in the first biological reactor 500, over 2000 in the second biological reactor 510. Optionally, P is fully depleted in the second biological reactors in order to strengthen limitation condition on the biomass. Both reactors 500 and 510 are operated under aerobic and non-axenic conditions. They can be both of the continuous or semi-continuous type.

The PHA-rich biomass 180 produced in the second biological reactor is directed to a liquid-solid separator 360. The liquid phase 190 is redirected to the wastewater treatment line while the solid phase 370 made of PHA-rich biomass is sent to a DSP unit 700, wherein the PHA-rich biomass is dried in order to remove any trace of the cultivation liquid. The PHA is the recovered by any conventional method from the dried PHA-rich biomass 710.

Submitting the PHA-rich biomass produced in the first biological reactor once again to the method of the invention allows the maximisation of the PHA content in cells by imposing stronger double-limitation conditions. It helps as well to modify the PHA composition in order to customize the polymer produced according to the needs and requirements of a manufacturer. In particular, it is possible during this second stage to fix culture conditions in order to maximize the hydroxyvalerate HV content in cells.

### 6. Alternatives

In an alternative a third stage can be operated downstream the second stage. The second biological reactor is then connected to a third continuous and biological reactor, which is of the same type as first and second continuous and biological reactor. However, the third reactor would be of a similar or large volume than the second reactor. Then, the cell growth rate µ₃ fixed in reactor 3 is lower than µ₂.

In the second or third reactor, a nutrient starvation can be imposed (no residual growth), the cell residence time will then be defined based on the PHA cell content targeted and the PHA storage rate.

In another variant all the biological reactors are each connected to a liquid-solid separator. Alternatively, only the last biological reactor is coupled with a liquid-solid separator.

These successive steps aim at increasing step-wise the selection pressure on the PHA storing microorganisms. Therefore, a restricted consortium of high PHA storing strains is selected. Furthermore, the selection pressure is such that the entire carbon uptake by the biomass is directed to PHA production and storing.

The various stages enables:
- increasing further the PHA content in cells, and
- the manipulation of the polymer chemical composition in order to influence the synthesis ratio of different monomers of PHA.

More particularly, different configurations of two-stage system are possible, according to the final objective of the process and the applied culture conditions.

A first configuration of a two-stage water treatment plant consists in a first continuous and mixed biological reactor wherein the selection stage and production stage are achieved in the same time, as previously described. Preferably, the limitation is strong in order to achieve PHA content of 65% and more. The PHA producing biomass is then transferred to a second mixed biological reactor, wherein P is completely depleted or absent and the growth rate µ₂ is lower than the one in the first reactor in order to maximise the PHA content in cells. Liquid-solid separator can be used in order to regulate the growth rate between the reactors. Moreover, the inventors found out that, in the presence of the appropriate precursors (propionate or valerate, VFA with odd number of carbon atoms), the more the P limitation is strong, the more the microorganisms tend to synthetize hydroxyvalerate (HV) relative to hydroxybutyrate (HB) monomers, increasing the fraction of HV monomer in co-polymers composed of the two monomer types HB and HV, P(HB-co-HV). Such co-polymers have improved mechanical properties over the PHB homopolymer, and their thermal and mechanical properties vary with HV fraction. Therefore, this configuration helps to maximize the PHA content in cells and to control the final composition of the PHA produced by the biomass (eg. maximise the HV fraction of the PHA).

A second configuration of a two-stage water treatment plant consists in a first continuous and mixed biological reactor wherein the selection stage and production stage are achieved in the same time, as previously described in example 1. However, the applied limitation is not very strong in order to achieve only PHA content in cells of 25%-30%. The PHA producing biomass is then transferred to a second mixed biological reactor, wherein P limitation is stronger and the cell specific growth rate µ₂ is lower than the one in the first reactor in order to maximise the PHA content in cells (around 65% or more). This configuration may be preferred in order to avoid a washout in the first biological reactor.

A three-stage water treatment combines the advantages of these two previous configurations: the P limitation can be mild in the first biological reactor, strong in the second reactor and P can be absent from the third reactor. Then, the PHA content increases as the biomass passes from a reactor to the following one. Moreover, the final composition of produced PHA is enriched in HV, thanks to the progressive P limitation.

When carried out in two or three stages, all the biological reactors can be operated either in a continuous or semi-continuous mode. Alternatively, only the first biological reactor is operated in a continuous or semi-continuous mode while the second and the third one(s) can be operated either in a continuous, semi-continuous, batch or fed-batch mode.

The method of the invention can also be carried out with other nutrient limitations than phosphorus.

### 7. Conclusion

The method according to the invention enables the selection of only high producing microorganisms from a natural source comprising a multiplicity of bacterial strains. Therefore, it is not necessary to work under restricting and sterile conditions.

Contrarily to the method of the prior art, culture selection and PHA production are carried out in a single step, which can be carried out either in a single stage, in a single continuous and mixed biological reactor, or in several consecutive stages at increasing limitation degree. The cultivation parameters are carefully chosen and regulated to create an unbalance between the carbon source uptake by the bacteria and the carbon requirements from the imposed cell growth, which is controlled through the fixed specific cell growth rate and through an imposed nutrient limitation. The unbalance between the carbon source uptake and cell growth (while maintaining a certain growth) consists in the optimal condition for PHA production and storing (maximum PHA yield on substrate - close to theoretical maximum - and maximum PHA storage rates). Therefore, the global efficiency of the process is improved in comparison to mixed culture methods of the prior art wherein the PHA yield on substrate is less favourable (given the carbon required for the selection step in which PHA is stored and re-consumed). It should also be said that relative to pure culture systems it represents a similar functional stability at significantly lower operating costs.

Therefore, the method of the invention enables high selection efficiency and high PHA storage performance at low cost. In other words, the method of the invention allows the combination of the advantages of pure and mixed culture systems, without their respective inconvenient.

### 8. References

1. Lee, S.Y. (1996) Bacterial polyhydroxyalkanoates. Biotechnol. Bioeng. 49: 1-14
2. Crank, M., Patel, M., 2005. Techno-economic Feasibility of Largescale Production of Bio-based Polymers in Europe. European Commission.
3. Serafim LS, Lemos PC, Torres C, et al. The influence of process parameters on the characteristics of PHA produced by mixed cultures. Macromolecular Bioscience 2008, 8(4): 355-366.
4. M. Reis, M. Albuquerque, M. Villano, M. Majone. Mixed Culture Processes for Polyhydroxyalkanoate Production from Agro-Industrial Surplus/Wastes as Feedstocks. Comprehensive Biotechnology (Second Edition), 2011, 6 : 669-683
5. H. Salehizadeh, M.C.M. Van Loosdrecht. Production of polyhydroxyalkanoates by mixed culture: recent trends and biotechnological importance. Biotechnology Advances 2004, 22(3):261-279.
6. M.G.E. Albuquerque, C.A.V. Torres, M.A.M. Reis. Polyhydroxyalkanoate (PHA) production by a mixed microbial culture using sugar molasses: Effect of the influent substrate concentration on culture selection. Water Research 2010, 44 (11) :3419-3433.
7. Katja Johnson, Robbert Kleerebezem, Mark C.M. van Loosdrecht. Influence of the C/N ratio on the performance of polyhydroxybutyrate (PHB) producing sequencing batch reactors at short SRTs. Water Research 2010, 44 (7): 2141-2152.
8. Dionisi D, Beccari M, Di Gregorio S, et al. Storage of biodegradable polymers by an enriched microbial community in a sequencing batch reactor operated at high organic load rate. Journal of Chemical Technology and Biotechnology 2005, 80: 1306-1318.
9. Dionisi D, Majone M, Vallini G, et al. Effect of applied organic load rate on biodegradable polymer production by mixed microbial cultures in a sequencing batch reactor. Biotechnology and Bioengineering (2006); 93: 76-88.
10. Maria G E Albuquerque, Gilda Carvalho, Caroline Kragelund, Ana F Silva, Maria T Barreto Crespo, Maria A M Reis & Per H Nielsen et al. Link between microbial composition and carbon substrate-uptake preferences in a PHA-storing community. The ISME Journal 2012, 74.

## Claims

1. Method for selection and maintenance of a selected microbial consortium for polyhydroxyalkanoates (PHA) production, said microbial consortium being fed with at least one readily biodegradable carbon substrate, comprising a first step of aerobic cultivation of said microbial consortium in a mixed biological reactor;
**characterized in that** the specific cell growth rate µ₁ of said microbial consortium is fixed at a target value in said first step of aerobic cultivation, and **in that** said first step of aerobic cultivation is performed under nutrient limitation such as the readily biodegradable carbon substrate uptake rate qS₁ is unbalanced with said fixed specific cell growth rate µ₁ and **in that** said selection is carried out simultaneously and in the same reactor as said PHA production;
wherein the specific cell growth rate µ₁ of said microbial consortium is fixed between 0.01h⁻¹ and 0.1h⁻¹;
wherein said specific cell growth rate µ₁ does not vary more than 50% around the target value.

2. Method according to claim 1 wherein the ratio µ₁/qS₁ between the specific cell growth rate µ₁ and the readily biodegradable carbon substrate uptake rate qS₁ is comprised in a range between 0.01 and 0.5 gram of biomass produced per gram of readily biodegradable carbon substrate consumed.

3. Method according to claim 1 or 2 wherein the fixation of the specific cell growth rate µ₁, during the first aerobic cultivation step, is achieved by the control of the said microbial consortium's residence time in the mixed biological reactor.

4. Method according to any of the claims 1 to 3 wherein growth limitation is achieved by the limitation of at least one nutrient chosen among P, N, O, S, K, Mg, Fe or a combination thereof.

5. Method according to claims 1-4 wherein the limited nutrient is P and the P/C molar ratio is comprised between 0.0002 and 0.0005 when the specific fixed cell growth rate µ₁ is comprised between 0.01 h-¹ and 0.05 h⁻¹.

6. Method according to claims 1-4 wherein the P/C molar ratio is in the range of 0.0005 to 0.005 when the specific growth rate µ₁ is comprised between 0.05 h⁻¹ and 0.1 h⁻¹.

7. Method according to any of the preceding claims wherein said growth limitation consists in the separate addition of the readily biodegradable carbon substrate and the limiting nutrient.

8. Method according to any of the preceding claims further comprising a step of continuous monitoring of the PHA content and the PHA composition in said microbial consortium.

9. Method according to any of the preceding claims further comprising a step of monitoring the readily biodegradable carbon substrate residual concentration in the biological reactor.

10. Method according to any of the preceding claims, wherein the reactor further comprises a control loop triggered by a parameter chosen among the readily biodegradable carbon substrate accumulation in said continuous biological reactor, the decrease of PHA cell content, the decrease of PHA cell content to a predetermined value or the combination thereof.

11. Method according to any of the preceding claims wherein said first step of aerobic cultivation is carried out in a single stage in a mixed, continuous or semi-continuous, biological reactor.

12. Method according to claim 13 wherein said first step of aerobic cultivation further comprises at least one or more additional stage, each of said at additional stage being carried out under more severe nutrient limitation than the previous one, the specific cell growth rate of each stage being lower than the specific fixed cell growth rate of the previous one and each stage being carried out either in a mixed continuous, semi-continuous, batch or fed-batch biological reactor.

13. Method according to any of the preceding claims wherein the total COD of the readily biodegradable carbon substrate-rich effluent comprises at least 50% of RBCOD (Readily Biodegradable Chemical Oxygen Demand) substrate and at least 30% of VFA (Volatile Fatty Acids).

## Patentansprüche

1. Verfahren zur Auswahl und Beibehaltung eines ausgewählten mikrobiellen Konsortiums zur Herstellung von Polyhydroxyalkanoaten (PHA), wobei das mikrobielle Konsortium mit mindestens einem leicht biologisch abbaubaren Kohlenstoffsubstrat versorgt wird, umfassend einen ersten Schritt des aeroben Kultivierens des mikrobiellen Konsortiums in einem gemischten biologischen Reaktor,
**dadurch gekennzeichnet dass** die spezifische Zellwachstumsrate µ₁ des mikrobiellen Konsortiums auf einen Zielwert in dem ersten Schritt des aeroben Kultivierens fixiert ist, und dadurch, dass der erste Schritt des aeroben Kultivierens unter Nährstoffmangel durchgeführt wird, so dass die Aufnahmerate qS₁ des leicht biologisch abbaubaren Kohlenstoffsubstrats nicht mit der fixierten spezifischen Zellwachstumsrate µ₁ ausbalanciert ist, und dadurch, dass die Auswahl gleichzeitig und im gleichen Reaktor wie die PHA-Herstellung durchgeführt wird,
wobei die spezifische Zellwachstumsrate µ₁ des mikrobiellen Konsortiums zwischen 0,01 h⁻¹ und 0,1 h⁻¹ fixiert ist;
wobei die spezifische Zellwachstumsrate µ₁ nicht mehr als 50 % um den Zielwert variiert.

2. Verfahren nach Anspruch 1, wobei das Verhältnis µ_{1/}qS₁ zwischen der spezifischen Zellwachstumsrate µ₁ und der Aufnahmerate qS₁ des leicht biologisch abbaubaren Kohlenstoffsubstrats im Bereich zwischen 0,01 und 0,5 Gramm Biomasse, hergestellt pro Gramm verbrauchtem leicht biologisch abbaubarem Kohlenstoffsubstrat, liegt.

3. Verfahren nach Anspruch 1 oder 2, wobei die Fixierung der spezifischen Zellwachstumsrate µ₁ während des ersten aeroben Kultivierungsschritts durch die Steuerung der Aufenthaltszeit des mikrobiellen Konsortiums im gemischten biologischen Reaktor erzielt wird.

4. Verfahren nach einem der Ansprüche 1 bis 3, wobei die Wachstumsbegrenzung durch die Begrenzung von mindestens einem Nährstoff, ausgewählt aus P, N, O, S, K, Mg, Fe oder einer Kombination davon erzielt wird.

5. Verfahren nach Anspruch 1 - 4, wobei der begrenzte Nährstoff P ist und das Molarverhältnis P/C im Bereich zwischen 0,0002 und 0,0005 liegt, wenn die spezifische fixierte Zellwachstumsrate µ₁ im Bereich zwischen 0,01 h⁻¹ und 0,05 h⁻¹ liegt.

6. Verfahren nach Anspruch 1 - 4, wobei das Molarverhältnis P/C im Bereich von 0,0005 bis 0,005 liegt, wenn die spezifische fixierte Zellwachstumsrate µ₁ im Bereich zwischen 0,05 h⁻¹ und 0,1 h⁻¹ liegt.

7. Verfahren nach einem der vorhergehenden Ansprüche, wobei die Wachstumsbegrenzung aus der getrennten Zugabe des leicht biologisch abbaubaren Kohlenstoffsubstrats und des begrenzenden Nährstoffs besteht.

8. Verfahren nach einem der vorhergehenden Ansprüche, weiter umfassend einen Schritt des kontinuierlichen Überwachens des PHA-Gehalts und der PHA-Zusammensetzung in dem mikrobiellen Konsortium.

9. Verfahren nach einem der vorhergehenden Ansprüche, weiter umfassend einen Schritt des Überwachens der restlichen Konzentration des leicht biologisch abbaubaren Kohlenstoffsubstrats im biologischen Reaktor.

10. Verfahren nach einem der vorhergehenden Ansprüche, wobei der Reaktor weiter einen Regelkreis umfasst, der von einem Parameter ausgelöst wird, ausgewählt aus der Akkumulation des leicht biologisch abbaubaren Kohlenstoffsubstrats in dem kontinuierlichen biologischen Reaktor, der Abnahme des PHA-Zellengehalts, der Abnahme des PHA-Zellengehalts auf einen vorbestimmten Wert oder der Kombination davon.

11. Verfahren nach einem der vorhergehenden Ansprüche, wobei der erste Schritt des aeroben Kultivierens in einer einzigen Stufe in einem gemischten, kontinuierlichen oder halbkontinuierlichen biologischen Reaktor durchgeführt wird.

12. Verfahren nach Anspruch 13, wobei der erste Schritt des aeroben Kultivierens weiter mindestens einen oder mehrere zusätzliche Schritte umfasst, wobei jeder des mindestens einen zusätzlichen Schritts unter einer strengeren Nährstoffbeschränkung als der vorhergehende durchgeführt wird, wobei die spezifische Zellwachstumsrate jeder Stufe geringer als die spezifische fixierte Zellwachstumsrate des vorherigen ist und jede Stufe entweder in einem gemischten kontinuierlichen, halbkontinuierlichen Batch- oder Fed-Batch biologischem Reaktor durchgeführt wird.

13. Verfahren nach einem der vorhergehenden Ansprüche, wobei der gesamte COD des leicht biologisch abbaubaren Kohlenstoffsubstrat-reichen Ausflusses mindestens 50 % RBCOD (Readily Biodegradable Chemical Oxygen Demand) -Substrat und mindestens 30 % VFA (Volatile Fatty Acids) umfasst.

## Revendications

1. Procédé pour la sélection et la maintenance d'un consortium microbien sélectionné pour la production de polyhydroxyalcanoates (PHA), ledit consortium microbien étant alimenté avec au moins un substrat carboné facilement biodégradable, comprenant une première étape de culture aérobie dudit consortium microbien dans un réacteur biologique mixte ;
**caractérisé en ce que** le taux de croissance cellulaire spécifique µ₁ dudit consortium microbien est fixé à une valeur cible dans ladite première étape de culture aérobie, et **en ce que** ladite première étape de culture aérobie est effectuée avec limitation de nutriments de façon que le taux d'assimilation de substrat carboné facilement biodégradable qS₁ ne soit pas équilibré avec ledit taux de croissance cellulaire spécifique µ₁ fixé et **en ce que** ladite sélection est réalisée simultanément et dans le même réacteur que ladite production de PHA ;
dans lequel le taux de croissance cellulaire spécifique µ₁ dudit consortium microbien est fixé entre 0,01 h⁻¹ et 0,1 h⁻¹ ;
dans lequel ledit taux de croissance cellulaire spécifique µ₁ ne varie pas de plus de 50 % autour de la valeur cible.

2. Procédé selon la revendication 1 dans lequel le rapport µ₁/qS₁ entre le taux de croissance cellulaire spécifique µ₁ et le taux d'assimilation de substrat carboné facilement biodégradable qS₁ est compris dans une plage située entre 0,01 à 0,5 gramme de biomasse produite par gramme de substrat carboné facilement biodégradable consommé.

3. Procédé selon la revendication 1 ou 2 dans lequel la fixation du taux de croissance cellulaire spécifique µ₁, lors de la première étape de culture aérobie, est obtenue par le contrôle du temps de présence dudit consortium microbien dans le réacteur biologique mixte.

4. Procédé selon l'une quelconque des revendications 1 à 3 dans lequel la limitation de croissance est obtenue par la limitation d'au moins un nutriment choisi parmi P, N, O, S, K, Mg, Fe ou une combinaison de ceux-ci.

5. Procédé selon l'une quelconque des revendications 1 à 4 dans lequel le nutriment limité est P et le rapport molaire P/C est compris entre 0,0002 et 0,0005 quand le taux de croissance cellulaire fixé spécifique µ₁ est compris entre 0,01 h⁻¹ et 0,05 h⁻¹.

6. Procédé selon l'une quelconque des revendications 1 à 4 dans lequel le rapport molaire P/C est dans la plage allant de 0,0005 à 0,005 quand le taux de croissance spécifique µ₁ est compris entre 0,05 h⁻¹ et 0,1 h⁻¹.

7. Procédé selon l'une quelconque des revendications précédentes dans lequel ladite limitation de croissance consiste en l'ajout séparé du substrat carboné facilement biodégradable et du nutriment limitant.

8. Procédé selon l'une quelconque des revendications précédentes comprenant en outre une étape de surveillance continue de la teneur en PHA et de la composition des PHA dans ledit consortium microbien.

9. Procédé selon l'une quelconque des revendications précédentes comprenant en outre une étape de surveillance de la concentration résiduelle en substrat carboné facilement biodégradable dans le réacteur biologique.

10. Procédé selon l'une quelconque des revendications précédentes, dans lequel le réacteur comprend en outre une boucle de commande déclenchée par un paramètre choisi parmi l'accumulation du substrat carboné facilement biodégradable dans ledit réacteur biologique continu, la diminution de la teneur en PHA dans les cellules, la diminution de la teneur en PHA dans les cellules jusqu'à une valeur prédéterminée ou la combinaison de ceux-ci.

11. Procédé selon l'une quelconque des revendications précédentes dans lequel ladite première étape de culture aérobie est réalisée en une seule phase dans un réacteur biologique mixte, continu ou semi-continu.

12. Procédé selon la revendication 13 dans lequel ladite première étape de culture aérobie comprend en outre au moins une ou plusieurs phases supplémentaires, chacune desdites phases supplémentaires étant réalisées avec une limitation de nutriments plus sévère que la précédente, le taux de croissance cellulaire spécifique de chaque phase étant inférieur au taux de croissance cellulaire fixé spécifique de la précédente et chaque phase étant réalisée dans un réacteur biologique mixte continu, semi-continu, discontinu ou discontinu à alimentation programmée.

13. Procédé selon l'une quelconque des revendications précédentes dans lequel la COD totale de l'effluent riche en substrat carboné facilement biodégradable comprend au moins 50 % de substrat RBCOD (demande chimique en oxygène facilement biodégradable) et au moins 30 % de VFA (acides gras volatils).
